# EUROPÄISCHE PATENTSCHRIFT

(11) **EP 2 279 164 B1**
(45) Veröffentlichungstag und Bekanntmachung des Hinweises auf die Patenterteilung: **28.03.2012**
(21) Anmeldenummer: 09745709.7
(22) Anmeldetag: 11.05.2009
(51) Int. Cl.: C07C 211/11, C07C 209/60

(54) **VERFAHREN ZUR HERSTELLUNG VON N,N-SUBSTITUIERTEN-1,3-PROPANDIAMINEN**
METHOD FOR PRODUCING N,N-SUBSTITUTED-1,3-PROPANDIAMINES
PROCÉDÉ DE PRODUCTION DE 1,3-PROPANEDIAMINES À SUBSTITUTION N,N

(30) Priorität: 13.05.2008 EP 08156092; 05.11.2008 EP 08168393
(43) Veröffentlichungstag der Anmeldung: 02.02.2011
(73) Patentinhaber: BASF SE, 67056 Ludwigshafen (DE)
(72) Erfinder: ERNST, Martin, 69115 Heidelberg (DE); MELDER, Johann-Peter, 67459 Böhl-Iggelheim (DE); WIGBERS, Christof Wilhelm, 68167 Mannheim (DE)
(86) Internationale Anmeldenummer: PCT/EP2009/055655
(87) Internationale Veröffentlichungsnummer: WO 2009/138377

(56) Entgegenhaltungen:
- EP-A- 0 590 419
- WO-A-2004/060853
- US-A- 3 383 392
- US-B1- 6 660 887

## Beschreibung

Die vorliegende Erfindung betrifft ein Verfahren zur Herstellung von N,N-substituierten-1,3-propandiaminen.

N,N-substituierte-1,3-propandiamine werden üblicherweise durch Umsetzung von sekundären Aminen mit Acrylnitril und nachfolgender Hydrierung der Nitrilgruppe zum Amin erhalten. So wird beispielsweise das technisch bedeutende N,N-Dimethyl-1,3-propandiamin (DMAPA) auf Basis von Dimethylamin und Acrylnitril hergestellt. Der Ausgangsstoff Acrylnitril wird üblicherweise durch den SOHIO-Prozess erhalten, einer katalytisch gesteuerten Synthese von Propen mit Ammoniak und elementarem Sauerstoff. Dabei entsteht unter Abspaltung von Wasser Acrylnitril mit Acetonitril und Blausäure als Nebenprodukten.

Der Zugang zu chemischen Grundprodukten, wie Propen, aus nachwachsenden Rohstoffen ist in der Regel unökonomisch, da die von der Natur mit erheblichem Energieaufwand gebildeten komplexen Molekülstrukturen nur unter Energieaufwand in niedrigmolekulare Stoffe umgeformt werden können.

Ein niedermolekulares chemisches Grundprodukt, das allerdings kommerziell aus nachwachsenden Rohstoffen gewonnen werden kann, ist Acrolein, das durch Dehydratisierung von Glycerin hergestellt wird. Glycerin wiederum fällt üblicherweise als Nebenprodukt bei der Umwandlung von Fetten und Ölen zu Fettsäuren (Fettverseifung) oder Fettsäuremethylestern (Biodiesel) an.

Die Umsetzung von sekundären aliphatischen Aminen mit Acrolein wurde erstmalig von Mannich et al. beschrieben (C. Mannich, K. Handke und K. Roth, Chem. Ber., 69, 2112 (1936)). Durch Umsetzung von Dimethylamin mit Acrolein in Gegenwart eines wasserentziehenden Mittels oder in eines nicht mit Wasser mischbaren Lösungsmittels wurde in einem engen Druck- und Temperaturbereich N,N,N',N'-Tetramethyl-1,3-propendiamin erhalten, welches in einer weiteren Stufe durch katalytische Reduktion mit Wasserstoff an Platinoxid und Cyclohexan als Lösungsmittel weiter zum entsprechenden N,N,N',N'-Tetramethyl-1,3-propandiamin umgesetzt wurde. Offenbarungsgemäß muss das sich bei der Reaktion bildende Wasser aus dem Reaktionsgemisch entfernt werden, da die sich bildenden Produkte leicht hydrolysierbar sind. Die durch die von Mannich et al. beschriebene Reaktion erhaltenen N,N,N',N'-Tetramethyl-1,3-propandiamine werden zum Teil als Katalysatoren bei der Herstellung von Polyurethanen eingesetzt.

Ein Verfahren zur Umsetzung von primären aliphatischen oder cycloaliphatischen Aminen mit Acrolein zu N,N'-substituierten-1,3-Propendiaminen wird ebenfalls in US 2,565,488 bzw. in der äquivalenten DE-B-866647 offenbart. Die sich bei der Reaktion bildenden Wassermengen können offenbarungsgemäß in der Reaktionsmischung belassen werden. Wird die Reaktion jedoch in Anwesenheit von wasserentziehenden Mitteln (Trocknungsmitteln) durchgeführt, so werden höhere Ausbeuten an N,N'-substituierten-1,3-Propendiamin erzielt. Es wird beschrieben, dass N,N'-substituierte-1,3-Propendiamine mit Wasserstoff gesättigt werden können, so dass beispielsweise N,N'-substituierte-1,3-Propandiamine erhalten werden. Es wird weiterhin beschrieben, dass N,N'-substituierte-1,3-Propendiamine mit Aminen, die andere Substituenten enthalten, vor der Hydrierung in einem weiteren Reaktionsschritt umaminiert werden können.

DE-A1-4232424 betrifft ein Verfahren zur Herstellung von N,N'-substituierten bzw. N,N,N',N'-substituierten ungesättigen Aminen durch Umsetzung von 2-Alkenalen mit primären oder sekundären Aminen bei Temperaturen von (-20) bis 70°C und Drücken von 0,10 bis 100 bar, indem man die Umsetzung ohne Abtrennung des Reaktionswassers durchführt. Weiterhin beschreibt die Offenbarung die Umaminierung der so erhaltenen ungesättigten Amine durch deren Umsetzung mit anderen primären oder sekundären Aminen bei Temperaturen von 20 bis 150°C. Die in DE-A1-4232424 erhaltenen ungesättigten Amine können offenbarungsgemäß mit Wasserstoff bei einem Druck von 1 bis 350 bar und Temperaturen von 0 bis 150°C hydriert werden, wobei beispielsgemäß auf Aktivkohle geträgertes Palladium als Hydrierkatalysator eingesetzt wird.

Finch et al. (H. D. Finch, E. A. Peterson und S. A. Ballard, J. Am. Chem. Soc., 74, 2016 (1952)) beschreiben die Reaktion von Acrolein bzw. Methacrolein mit primären oder sekundären Aminen. Die so erhaltenen N,N'-substituierten-1,3-Propendiamine bzw. N,N,N',N'-substituierten-1,3-Propendiamine werden offenbarungsgemäß in einer nachfolgenden Stufe zu den korrespondierenden gesättigten N,N'-substituierten- bzw. N,N,N',N'-substituierten-1,3-Propandiaminen hydriert oder zusammen mit anderen Aminen erhitzt, so dass ein Aminaustausch stattfindet. Es wird beschrieben, dass Aminaustausch und Hydrierung auch gleichzeitig in Gegenwart eines Raney®-NickelKatalysators durchgeführt werden können. So wurde N-Isopropyl-1,3-propandiamin in zwei Stufen erhalten, wobei in der ersten Stufe zunächst Acrolein und Isopropylamin umgesetzt wurde und in einer zweiten Stufe die erhaltene Reaktionsmischung nach Entfernung von überschüssigem Amin und Lösungsmittel mit Ammoniak in Gegenwart von Raney®-Nickel hydriert wurde. Die Ausbeuten an N-Isopropyl-1,3-propandiamin, bezogen auf das in die Reaktion eingesetzte Acrolein, betrugen weniger als 68%.

Die Aufgabe der vorliegenden Erfindung bestand in der Bereitstellung eines Verfahrens zur Herstellung von N,N-substituierten-1,3-propandiaminen aus Acrolein, wobei gegenüber dem Stand der Technik eine höhere Selektivität bezogen auf das eingesetzte Acrolein erzielt werden sollte. Es war insbesondere Aufgabe die Bildung von Nebenprodukten, wie 3-Methylpiperidin und N,N,N',N'-substiuierten-1,3-propandiaminen, die bei der Hydrierung der intermediär auftretenden N,N,N',N'-substituierten-1,3-propendiamine entstehen können, zu verringern. Weiterhin war es ein Ziel ein Verfahren zur Verfügung zu stellen, bei dem die als Zwischenstufe erhaltenen N,N,N',N'-substituierten-1,3-propendiamine vor der weiteren Umsetzung zu N,N-substituierten-1,3-propandiamin nicht isoliert bzw. aufgereinigt werden müssen. Somit sollte ein leicht zu handhabendes Verfahren realisiert werden, dass technisch einfach umgesetzt werden kann. Insbesondere war es ein Ziel der vorliegenden Erfindung eine neue Herstellroute für DMAPA zur Verfügung zu stellen, in der Einsatzstoffe verwendet werden, die auf Basis nachwachsender Rohstoffe erhalten werden können.

Erfindungsgemäß wurde die Aufgabe durch ein Verfahren zur Herstellung von N,N-substiuierten-1,3-propandiamin durch
a) Umsetzung von sekundären Amin mit Acrolein bei einer Temperatur von (-50) bis 100°C und einem Druck von 0,01 bis 300 bar, und
b) Umsetzung des in Stufe a) erhaltenen Reaktionsgemischs mit Wasserstoff und Ammoniak in Gegenwart eines Hydrierkatalysators bei einer Temperatur von 40 bis 400°C und einem Druck von 1 bis 400 bar,
dadurch gekennzeichnet, dass in Stufe a) das molare Verhältnis von sekundärem Amin zu Acrolein 2:1 oder mehr beträgt und der in Stufe b) eingesetzte Hydrierkatalysator Kobalt enthält, gelöst.

In der ersten Stufe a) des erfindungsgemäßen Verfahrens wird Acrolein mit sekundärem Amin umgesetzt.

Das in die Reaktion eingesetzt Acrolein wird üblicherweise durch Oxidation von Propen oder durch Dehydratisierung von Glycerin erhalten.

Üblicherweise wird Acrolein durch Oxidation von Propen hergestellt. Eine Übersicht über die Acrolein-Herstellung durch Propen-Oxidation findet man beispielsweise im Ullmann (Ullmann's Encyclopedia of Industrial Chemistry, Acrolein and Methacrolein, Kapitel 3.1 "Acrolein by Propene Oxidation", Wiley-VCH-Verlag, Electronic Edition, 2007).

In einer bevorzugten Ausführungsform wird Acrolein eingesetzt, dass durch Dehydratisierung von Glycerin erhalten wurde. Die Herstellung von Acrolein durch Dehydratisierung aus Glycerin wird beispielsweise in WO-A2-2006087083, EP-B1-598228, WO-A1-2007090990, US 5,079,266, US 2,558,520 oder von Chai et al. (S. H. Chai, H. P. Wang, Y. Lang, B. Q. Xu, Journal of Catalysis, 250 (2), 342-349 (2007)) offenbart. Glycerin fällt üblicherweise als Nebenprodukt bei der Umwandlung von Fetten und Ölen zu Fettsäuren (Fettverseifung) oder Fettsäuremethylestern (Biodiesel) an. Die Herstellung von Glycerin aus Fetten und Ölen ist beispielsweise im Ullmann beschrieben (Ullmann's Encyclopedia of Industrial Chemistry, Glycerol, Kapitel 4.1 "Glycerol from Fat and Oils", Wiley-VCH-Verlag, Electronic Edition, 2007).

Glycerin kann auch ausgehend von dem petrochemischen Ausgangsprodukt Propen hergestellt werden. Ein Überblick über die Synthese von Glycerin aus Propen erfolgt ebenfalls im Ullmann (Ullmann's Encyclopedia of Industrial Chemistry, "Glycerol", Kapitel 4.1 "Synthesis from Propene", Wiley-VCH-Verlag, Electronic Edition, 2007).

Für das erfindungsgemäße Verfahren ist es in der Regel unerheblich auf welchem Herstellungsweg Glycerin erhalten wurde. Sowohl Glycerin auf pflanzlicher, tierischer oder petrochemischer Basis ist als Ausgangsstoff für das erfindungsgemäße Verfahren geeignet.

In einer ganz besonders bevorzugten Ausführungsform wird Glycerin auf Basis nachwachsender Rohstoffe als Ausgangsprodukt bei der Herstellung von Acrolein eingesetzt, beispielsweise Glycerin, das als Nebenprodukt aus der Fettverseifung bzw. Biodieselherstellung anfällt. Diese besondere Ausführungsform hat den Vorteil, dass technisch bedeutende Amine, wie DMAPA, aus erneuerbaren Ressourcen erhalten werden können. Der Einsatz von erneuerbaren Ressourcen bei der Herstellung solcher Produkte kann zu einer Schonung der üblicherweise eingesetzten petrochemischen Ressourcen beitragen.

Bevorzugt wird im erfindungsgemäßen Verfahren Acrolein mit einem Acrolein-Gehalt von mindestens 95%, bevorzugt mindestens 98% und besonders bevorzugt mindestens 99% eingesetzt.

Als weiterer Einsatzstoff wird im erfindungsgemäßen Verfahren sekundäres Amin eingesetzt.

Als sekundäres Amin können aliphatische, cycloaliphatische oder cyclische sekundäre Amine eingesetzt werden.

Als cyclisches sekundäres Amin kann beispielsweise Pyrolidin, Imidazol, Piperidin, Morpholin oder Piperazin eingesetzt werden.

Vorzugsweise werden sekundäre Amine der Formel (I) 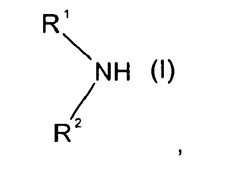 wobei die Reste R¹ und R² folgende Bedeutung haben:
R¹ und R² sind gleichzeitig oder unabhängig voneinander ein geradkettiger oder verzweigter oder ein cyclischer Kohlenwasserstoffrest mit 1 bzw. 3 bis 20 C-Atomen, wobei der Kohlenstoffrest einfach oder mehrfach ungesättigt sein kann.

Beispielsweise können R¹ und/oder R² folgende Bedeutung besitzen:
C₁-C₆-Alkyl: z.B. Methyl, Ethyl, n-Propyl, 1-Methylethyl, n-Butyl, 1-Methylpropyl, 2-Methylpropyl, 1,1-Dimethylethyl n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl oder 1-Ethyl-3-methylpropyl;
C₁-C₁₂-Alkyl: z.B. C₁-C₆-Alkyl, wie voranstehend genannt, sowie Heptyl, 2-Methylhexyl, 3-Methylhexyl, 2,2-Dimethylpentyl, 2,3-Dimethylpentyl, 2,4-Dimethylpentyl, 3,3-Dimethylpentyl, 2,2-Dimethyl-3-methylbutyl, Octyl, 2-Methylheptyl, 3-Methylheptyl, 4-Methylheptyl, 2,2-Diemthylhexyl, 2,3-Dimethylhexyl, 2,4-Dimethylhexyl, 3,3-Dimethylhexyl, 2,2-Dimethyl-3-methylpentyl, 2-Methyl-3,3-dimethylpentyl, 2,3,4-Trimethylpentyl und 2,2,3,3-Tetramethylbutyl, 1-Nonyl, 1-Decyl, 1-Undecyl oder1-Dodecyl;
C₁-C₂₀-Alkyl: z.B. C₁-C₁₂-Alkyl, wie voranstehend genannt, sowie 1-Tridecyl, 1-Tetradecyl, 1-Pentadecyl, 1-Hexadecyl, 1-Heptadecyl, 1-Octadecyl, Nonadecyl oder Eicosyl;
C₃- bis C₈-Cycloalkyl: z.B. Cyclopropyl, Cyclobutyl, Cyclopentyl, Cyclohexyl oder Cyclooctyl;
C₃- bis C₁₂-Cycloalkyl: C₃- bis C₈-Cycloalkyl wie voranstehend genannt, sowie Cyclododecyl;
C₂-C₆-Alkenyl: z.B. Ethenyl, Propenyl, Butenyl, Pentenyl oder Hexenyl;
C₂-C₂₀-Alkenyl: z.B. C₂-C₆-Alkenyl wie voranstehend genannt, sowie Hepteny, Octenyl, Nonenyl oder Decenyl;
C₃-C₆-Cycloalkenyl: z.B. Cyclopropenyl, Cyclobutenyl, Cyclopentenyl, oder Cyclohexenyl;
Aryl: ein- bis dreikerniger aromatischer Carbocyclus mit 6 bis 14 Ringgliedern, wie z.B. Phenyl, Naphthyl oder Anthracenyl;
Heteroaryl: z.B. Thienyl, Furyl, Pyrazolyl, Imidazolyl, Thiazolyl und Oxazolyl; oder C₇-C₁₂-Aralkyl wie z.B. Phenylmethyl, 1-Phenylethyl 2-Phenylethyl, 1-Phenylpropyl, 2-Phenylpropyl oder 3-Phenylpropyl.

Die Reste R¹ und R² können gegebenenfalls substituiert sein, wobei die Substituenten in einem breiten Bereich variiert werden können.

Bevorzugt sind R¹ und R² gleichzeitig oder unabhängig voneinander ein geradkettiger oder verzweigter Kohlenwasserstoffrest mit 1 bzw. 3 bis 20 C-Atomen, wobei der Kohlenstoffrest gesättigt ist.

Werden sekundäre Amine der Formel (I) eingesetzt, deren Substituenten R¹ und/oder R² ungesättigte Bindungen enthalten, so kann auch eine Hydrierung dieser Substituenten eintreten.

Vorzugsweise werden Dimethylamin, Diethylamin, Di-n-Propylamin, Di-Isopropylamin, Isopropylethylamin, Di-n-Butylamin, Di-s-Butylamin oder Di-Cyclohexylamin als sekundäre Amine in das Verfahren eingesetzt.

Besonders bevorzugt werden Dimethylamin oder Diethylamin, insbesondere bevorzugt Dimethylamin, in die Reaktion eingesetzt.

Das molare Verhältnis von Acrolein zu sekundären Amin beträgt erfindungsgemäß 2:1 oder mehr. In der Regel beträgt das molare Verhältnis von sekundärem Amin zu Acrolein im Bereich von 2:1 bis 50:1, bevorzugt von 2:1 bis 10:1, besonders bevorzugt von 2:1 bis 5:1.

Die Umsetzung von Acrolein mit sekundärem Amin kann ohne Katalysator oder in Gegenwart eines Katalysators erfolgen.

Als Katalysatoren kommen beispielsweise feste Brönstedt- oder Lewis-Säuren in Betracht, wie sie beispielsweise in der EP-A1-449089 (Seite 2, Spalte 2, Zeilen 11-20) und in dem Artikel von Tanabe et al. (K. Tanabe, Studies in Surface Science and Catalysis, Vol. 51, 1989,. S. 1 ff) beschrieben sind. Beispielhaft seien hier acide Metalloxidkatalysatoren, wie Aluminiumoxid, Titandioxid, Zirkoniumdioxid und Siliciumdioxid genannt. Weiterhin kommen mit Ammonium-Ionen beladene anorganische oder organische Ionenaustauscher, wie Zeolithe oder sulfonierte Copolymerisate aus Styrol und Divenylbenzol (z.B. der Marken Lewatit® der Fa. Lanxess, Amberlite® der Fa. Rohm & Haas) oder Ionenaustauscher auf Basis Siloxan (z.B. der Marke Deloxan® der Fa. Degussa) in Betracht.

Die Umsetzung von Acrolein mit sekundären Amin kann in Anwesenheit eines Lösungsmittels erfolgen, z.B. in Ether, wie Methyltertbutylether, Ethyltertbutylether oder Tetrahydrofuran (THF); Alkoholen, wie Methanol, Ethanol oder Isopropanol; Kohlenwasserstoffen, wie Hexan, Heptan oder Raffinatschnitte; Aromaten, wie Toluol; Amide, wie Dimethylformamid oder Dimethylacetamid oder Lactamen, wie N-Methylpyrrolidon, N-Ethylpryrrolidon, N-Methylcaprolactam oder N-Ethylcaprolactam. Als Lösungsmittel kommen auch geeignete Mischungen der zuvor aufgeführten Lösungsmittel in Betracht. Ein bevorzugtes Lösungsmittel ist THF. Das Lösungsmittel kann in einem Anteil von 5 bis 95 % Gew.-%, bevorzugt 20 bis 70 %, besonders bevorzugt 30 bis 60 %, eingesetzt werden, jeweils bezogen auf das Gesamtgewicht des Reaktionsgemisches, wobei das Gesamtgewicht des Reaktionsgemisches aus der Summe der Massen der in das Verfahren eingesetzten Ausgangsstoffen und Lösungsmittel ist.

Bevorzugt wird die Umsetzung von Acrolein mit sekundären Amin ohne einen Zusatz von Lösungsmittel durchgeführt.

Acrolein wird mit sekundären Amin bei einer Temperaturen von (-50) bis 100°C, bevorzugt (-20) bis 70°C, besonders bevorzugt (-10) bis 40°C, und Drücken von 0,01 bis 300 bar, beorzugt 0,1 bis 200 , besonders bevorzugt 1 bis 200 bar, ganz besonders bevorzugt Normaldruck (Atmosphärendruck) umgesetzt. Bei gasförmigen sekundären Aminen, wie Dimethylamin, wird die Umsetzung bevorzugt bei Drücken von 5 bis 400 bar, besonders bevorzugt 10 bis 300 bar, insbesondere 15 bis 200 bar, durchgeführt.

Die Umsetzung von Acrolein mit sekundären Amin kann entweder diskontinuierlich oder kontinuierlich durchgeführt werden.

Die diskontinuierliche Umsetzung von Acrolein mit sekundären Amin kann beispielsweise in einem Rührautoklaven, einer Blasensäule oder einem Umlaufreaktor, wie etwa einem Strahlschlaufenreaktor, erfolgen.

Bei der diskontinuierlichen Umsetzung von Acrolein mit sekundären Amin wird üblicherweise sekundäres Amin bzw. eine Suspension von sekundärem Amin und Katalysator und ggf. Lösungsmittel im Reaktor vorgelegt. Um einen hohen Umsatz und hohe Selektivität zu gewährleisten, wird die Suspension von sekundärem Amin und Katalysator mit Acrolein üblicherweise gut gemischt, z.B. durch einen Turbinenrührer in einem Autoklaven.

Das suspendierte Katalysatormaterial kann mit Hilfe gebräuchlicher Techniken eingebracht und wieder abgetrennt werden (Sedimentation, Zentrifugation, Kuchenfiltration, Querstromfiltration). Der Katalysator kann einmal oder mehrmals verwendet werden. Erfolgt die Umsetzung von Acrolein mit sekundären Amin in Gegenwart eines Katalysators, so beträgt die Katalysatorkonzentration vorteilhafter Weise 0,1 bis 50 Gew.-%, bevorzugt 0,5 bis 40 Gew.-%, besonders bevorzugt 1 bis 30 Gew.-%, insbesondere 5 bis 20 Gew.-%, jeweils bezogen auf das Gesamtgewicht der Suspension bestehend aus sekundärem Amin und Katalysator.

Die mittlere Katalysatorpartikelgröße liegt vorteilhaft im Bereich von 0,001 bis 1 mm, bevorzugt im Bereich von 0,005 bis 0,5 mm, insbesondere 0,01 bis 0,25 mm.

Die Umsetzung von Acrolein mit sekundären Amin wird bevorzugt kontinuierlich durchgeführt, üblicherweise in Druckbehältern oder Druckbehälterkaskaden.

Bevorzugt wird Acrolein und sekundäres Amin durch einen Rohrreaktor geleitet, in dem der Katalysator in Form eines Festbetts angeordnet ist.

In der Regel werden Acrolein und das sekundäre Amin vor der Einleitung in den Druckbehälter oder im Druckbehälter gut durchmischt. Die Durchmischung kann beispielsweise vor der Einleitung unter Verwendung von statischen Mischern erfolgen. In den Druckbehälter können auch Einbauten oder Mischelemente eingebracht sein, die die Mischung von Acrolein und sekundären Amin verbessern. Eine Durchmischung kann ggf. auch mittels eingebauten Rührern oder durch Umpumpen des Reaktionsgemisches erfolgen.

Vorzugsweise stellt man bei der kontinuierlichen Umsetzung von Acrolein mit sekundären Amin eine Katalysatorbelastung von 0,01 bis 10, vorzugsweise von 0,05 bis 7, besonders bevorzugt von 0,1 bis 5 kg Acrolein pro kg Katalysator und Stunde ein.

Das in Stufe a) erhaltene Reaktionsgemisch enthält N,N,N',N'-substituiertes-1,3-propendiamin.

Das in Stufe a) erhaltene Reaktionsgemisch kann vor Einsatz in Stufe b) aufgearbeitet werden, um das N,N,N',N'-substiuierte-1,3-propendiamin aufzukonzentrieren, beispielsweise durch Destillation oder Rektifikation.

Das in Stufe a) erhaltene Reaktionsgemisch wird jedoch bevorzugt vor Einsatz in Stufe b) ohne zusätzliche Aufreinigung oder Aufarbeitung eingesetzt.

In Stufe b) wird das in Stufe a) erhaltene Reaktionsgemisch mit Wasserstoff und Ammoniak in Gegenwart eines Hydrierkatalysators umgesetzt.

In das erfindungsgemäße Verfahren wird Wasserstoff eingesetzt. Der Wasserstoff kommt im Allgemeinen technisch rein zum Einsatz. Der Wasserstoff kann auch in Form eines Wasserstoff enthaltenden Gases, d.h. in Beimengungen mit anderen Inertgasen, wie Stickstoff, Helium, Neon, Argon oder Kohlendioxid zum Einsatz kommen. Als Wasserstoff enthaltende Gase können beispielsweise Reformerabgase, Raffineriegase usw. verwendet werden, wenn und soweit diese Gase keine Kontaktgifte für die eingesetzten Hydrierkatalysatoren, wie zum Beispiel CO enthalten. Bevorzugt wird jedoch reiner Wasserstoff bzw. im Wesentlichen reiner Wasserstoff in das Verfahren eingesetzt, beispielsweise Wasserstoff mit einem Gehalt von mehr als 99 Gew.-% Wasserstoff, bevorzugt mehr als 99,9 Gew.-% Wasserstoff, besonders bevorzugt mehr als 99,99 Gew.-% Wasserstoff, insbesondere mehr als 99,999 Gew.-% Wasserstoff.

In das erfindungsgemäße Verfahren wird weiterhin Ammoniak eingesetzt. Als Ammoniak kann herkömmlich im Handel erhältliches Ammoniak eingesetzt werden, beispielsweise Ammoniak mit einem Gehalt von mehr 98 Gew.-% Ammoniak, bevorzugt mehr als 99 Gew.-% Ammoniak, bevorzugt mehr als 99,5 Gew.-%, insbesondere mehr als 99,9 Gew.-% Ammoniak.

Das molare Verhältnis des in Stufe b) eingesetzten Ammoniaks zu dem in Stufe a) eingesetzten Acrolein beträgt vorzugsweise 1:1 bis 1000:1, vorzugsweise 2:1 bis 100:1, besonders bevorzugt 4:1 bis 50:1

In einer besonderen Ausführungsform wird die Stufe b) in Gegenwart von Wasser durchgeführt.

Die Menge Wasser wird bevorzugt so gewählt, dass das molare Verhältnis von Wasser zu Acrolein, das in Stufe a) eingesetzt wurde, im Bereich von 0,01:1 bis 100:1, vorzugweise im Bereich von 0,1:1 bis 50:1, besonders bevorzugt im Bereich von 0,5:1 bis 10:1 und ganz besonders bevorzugt im Bereich von 1:1 bis 5:1 liegt.

Das Wasser und das in Stufe a) erhaltene Reaktionsgemisch können gemeinsam in die Stufe b) gegeben werden, zum Beispiel als vorgemischter Reaktandenstrom, oder getrennt. Bei einer getrennten Zugabe können Wasser und das in Stufe a) erhaltene Reaktionsgemisch entweder gleichzeitig, zeitversetzt oder nacheinander in die Stufe b) gegeben werden. Es ist auch möglich, dass die Zugabe von Wasser bereits vor der Durchführung der Stufe a) durchgeführt wird und bereits in der Stufe a) zugegen ist, da die Anwesenheit von Wasser die Stufe a) nicht wesentlich beeinflusst. Vorzugsweise wird das Wasser jedoch erst vor Beginn der Stufe b) zugegeben.

Das erfindungsgemäße Verfahren wird in Gegenwart eines Hydrierkatalysators durchgeführt, welcher Kobalt enthält.

Der Hydrierkatalysator kann in metallischer Form in das Verfahren eingesetzt werden.

Kobalt (Co) kann in metallischer Form, beispielsweise als Metallnetz oder Metallgitter, als Hydrierkatalysator eingesetzt werden.

In einer bevorzugten Ausführungsform wird Co in metallischer Form als Schwamm-oder Skelettkatalysatoren nach Raney als Hydrierkatalysator in das erfindungsgemäße Verfahren eingesetzt.

Die Herstellung von Kobalt-Hydrierkatalysatoren nach Raney erfolgt üblicherweise durch Behandlung einer Aluminium-Kobalt-Legierung mit konzentrierter Natronlauge, wobei das Aluminium ausgelaugt wird und ein metallischer Kobaltschwamm entsteht. Die Herstellung von Hydrierkatalysatoren nach Raney ist beispielsweise im Handbook of Heterogeneous Catalysis beschrieben (M. S. Wainright in G. Ertl, H. Knözinger, J. Weitkamp (eds.), Handbook of Heterogeneous Catalysis, Vol. 1, Wiley-VCH, Weinheim, Germany 1997, Seite 64 ff.). Solche Katalysatoren sind beispielsweise als Raney®-Katalysatoren von der Fa. Grace oder als Sponge Metal®-Katalysatoren der Johnson Matthey erhältlich.

Bevorzugt beträgt der molare Anteil an Co-Atomen bezogen auf die Summe aller Metallatome im eingesetzten Hydrierkatalysator, der in metallischer Form in das Verfahren eingesetzt wurde, 50 Mol % und mehr, besonders bevorzugt 75 Mol %, ganz besonders bevorzugt 90 Mol % und mehr, insbesondere bevorzugt 99 Mol % und mehr. Die Zusammensetzung des Hydrierkatalysators, der in metallischer Form eingesetzt wird, kann mittels Atomabsorptionsspektrometrie (AAS), Atomemissionsspektrometrie (AES), Röntgenfluoreszenzanalyse (RFA) oder ICP-OES (Inductively Coupled Plasma Optical Emission Spectrometry) gemessen werden.

Die in das erfindungsgemäße Verfahren einsetzbaren Hydrierkatalysatoren können auch durch Reduktion von sogenannten Katalysatorvorläufern hergestellt werden.

Der Katalysatorvorläufer enthält eine aktive Masse, die eine oder mehrere katalytisch aktive Komponenten und optional ein Trägermaterial enthält.

Bei den katalytisch aktiven Komponenten handelt es sich um sauerstoffhaltige Verbindungen der Metalle der Gruppen 8 und/oder 9 und/oder 10 und/oder 11 des Periodensystems der Elemente (Periodensystem in der IUPAC-Fassung vom 22.06.2007), beispielsweise um deren Metalloxide bzw. Hydroxide. (ggf. Beispiele), wie CoO, NiO, Mn₃O₄, CuO, RuO(OH)ₓ und/oder deren Mischoxide.

Erfindungsgemäß enthält der Katalysatorvorläufer des Hydrierkatalysators sauerstoffhaltige Verbindungen des Kobalts als katalytisch aktive Komponente, beispielsweise als CoO und/oder als Mischoxid des Kobalts, wie LiCoO₂ Vorzugsweise enthält der Katalysatorvorläufer des Hydrierkatalysators CoO als katalytisch aktive Komponente. Neben sauerstoffhaltigen Verbindungen des Kobalts kann der Katalysatorvorläufer des Hydrierkatalysators weitere katalytisch aktive Komponenten enthalten.

Die im Folgenden angegebenen Zusammensetzungen beziehen sich auf die Zusammensetzung des Katalysatorvorläufers nach dessen letzter Wärmebehandlung, welche im Allgemeinen eine Calcinierung und/oder eine Temperung darstellt, und vor dessen Reduktion mit Wasserstoff.

Die Masse der aktiven Masse ist die Summe aus der Masse des Trägermaterials und aus der Masse der katalytisch aktiven Komponenten, d.h. dass das Trägermaterial als zur aktiven Masse gehörig gewertet wird.

Der molare Anteil an Co-Atomen bezogen auf die Summe aller Metallatome, die in den eingesetzten katalytisch aktiven Komponenten vorhanden sind, beträgt bevorzugt 10 Mol % oder mehr, besonders bevorzugt 30 Mol % oder mehr, ganz besonders bevorzugt 50 Mol % und mehr, insbesondere 90 Mol % und mehr.

Die atomare Zusammensetzung der katalytisch aktiven Komponenten kann mittels Atomabsorptionsspektrometrie (AAS), Atomemissionsspektrometrie (AES), Röntgenfluoreszenzanalyse (RFA) oder ICP-OES (Inductively Coupled Plasma Optical Emission Spectrometry) gemessen werden.

Der Anteil der aktiven Masse bezogen auf die Gesamtmasse des Katalysatorvorläufers beträgt üblicherweise 70 Gew.-% oder mehr, bevorzugt 80 bis 100 Gew.-%, besonders bevorzugt 90 bis 99 Gew.-%, insbesondere 92 bis 98 Gew.-%.

Der Katalysatorvorläufer kann neben der aktiven Masse weitere Komponenten, wie Verformungsmittel, beispielsweise Graphit, Stearinsäure, Phosphorsäure oder weitere Verarbeitungshilfsmittel enthalten.

Der Katalysatorvorläufer kann weiterhin als weitere Komponenten neben der aktiven Masse ein oder mehrere Dotierelemente (Oxidationsstufe 0) oder deren anorganische oder organische Verbindungen, ausgewählt aus den Gruppen 1 bis 14 des Periodensystems, enthalten. Beispiele für solche Elemente bzw. deren Verbindungen sind: Übergangsmetalle, wie Mn bzw. Manganoxide, Re bzw. Rheniumoxide, Cr bzw. Chromoxide, Mo bzw. Molybdänoxide, W bzw. Wolframoxide, Ta bzw. Tantaloxide, Nb bzw. Nioboxide oder Nioboxalat, V bzw. Vanadiumoxide bzw. Vanadylpyrophosphat, Zink bzw. Zinkoxide, Lanthanide, wie Ce bzw. CeO₂ oder Pr bzw. Pr₂O₃ Alkalimetalloxide, wie K₂O, Alkalimetallcarbonate, wie Na₂CO₃ und K₂CO₃, Erdalkalimetalloxide, wie SrO, Erdalkalimetallcarbonate, wie MgCO₃, CaCO₃, BaCO₃, Phosphorsäureanhydride und Boroxid (B₂O₃).

In einer bevorzugten Ausführungsform enthält die aktive Masse des Katalysatorvorläufers kein Trägermaterial.

Solche Katalysatorvorläufer sind beispielsweise in der Patenanmeldung mit dem Anmeldezeichen PCT/EP2007/052013 offenbarte Katalysatoren, die vor der Reduktion mit Wasserstoff a) Kobalt und b) ein oder mehrere Elemente der Alkalimetallgruppe, der Erdalkalimetallgruppe, der Gruppe der Seltenen Erden oder Zink oder Mischungen daraus enthalten, wobei die Elemente a) und b) zumindest zum Teil in Form ihrer Mischoxide vorliegen, beispielsweise LiCoO₂ oder

in EP-A-0636409 offenbarte Katalysatoren, deren katalytisch aktive Masse vor der Reduktion mit Wasserstoff 55 bis 98 Gew.-% Co, berechnet als CoO, 0,2 bis 15 Gew.-% Phosphor, berechnet als H₃PO₄, 0,2 bis 15 Gew.-% Mangan, berechnetl als MnO₂, und 0,2 bis 15 Gew.-% Alkali, berechnet als M₂O (M=Alkalimetall), enthält, oder

in EP-A-0742045 offenbarte Katalysatoren, deren katalytisch aktive Masse vor der Reduktion mit Wasserstoff 55 bis 98 Gew.-% Co, berechnet als CoO, 0,2 bis 15 Gew.-% Phosphor, berechnet als H₃PO₄, 0,2 bis 15 Gew.-% Mangan, berechnet als MnO₂, und 0,05 bis 5 Gew.-% Alkali, berechnet als M₂O (M=Alkalimetall), enthält.

Katalysatorvorläufer, die Mischoxide von Kobalt enthalten, wie LiCoO₂ und die bevorzugt kein Trägermaterial enthalten, lassen sich allgemein durch thermische Behandlung der entsprechenden Verbindungen des Kobalts und einem oder mehrerer Verbindungen der Alkalimetallgruppe, von Verbindungen der Erdalkalimetallgruppe, von Verbindungen aus der Gruppe der Seltenen Erden oder von Verbindungen des Zinks, beispielsweise den Nitraten, Carbonaten, Hydroxiden, Oxiden, Acetaten, Oxalaten oder Citraten herstellen. Thermische Behandlung kann beispielsweise als das Zusammenschmelzen oder Kalzinieren der oben genannten Verbindungen verstanden werden. Dabei kann die thermische Behandlung der oben genannten Verbindungen, wie den Nitraten, Carbonaten, Hydroxiden, Oxiden, an der Luft erfolgen. In einer bevorzugten Ausführungsform erfolgt die thermische Behandlung, insbesondere der Carbonate, unter einer Inertgas-Atmosphäre. Als Inertgas eignen sich z.B. Stickstoff, Kohlendioxid, Helium, Neon, Argon, Xenon, Krypton oder Mischungen der genannten Inertgase. Bevorzugt eignet sich Stickstoff.

Verfahren zur Herstellung von LiCoO₂ werden z.B. in Antolini [E. Antolini, Solid State Ionics, 159-171 (2004)] und Fenton et al. [W. M. Fenton, P. A. Huppert, Sheet Metal Industries, 25 (1948), 2255-2259) beschrieben.

Man kann als Katalysatorvorläufer, der bevorzugt kein Trägermaterial enthält, auch LiCoO₂ einsetzen, das durch die Wiederaufarbeitung von Bakterien gewonnen wird. Eine Methode zur Wiederverwertung bzw. Wiedergewinnung von Lithiumkobaltit aus Altbatterien kann beispielsweise aus CN-A-1594109 hergeleitet werden. Durch mechanisches Öffnen der Batterie und die Herauslösung von Aluminiumbestandteilen mit konz. NaOH kann ein LiCoO₂-reicher Filterkuchen erhalten werden.

In einer weiteren bevorzugten Ausführungsform enthält die aktive Masse - zusätzlich zu den katalytisch aktiven Komponenten - Trägermaterial.

Als Trägermaterial werden bevorzugt Kohlenstoff, wie Graphit, Russ und/oder Aktivkohle, Aluminiumoxid (gamma, delta, theta, alpha, kappa, chi oder Mischungen daraus), Siliziumdioxid, Zirkoniumdioxid, Zeolithe, Alumosilicate, etc. sowie Mischungen aus diesen Trägermaterialien verwendet.

Der Anteil an Trägermaterial an der aktiven Masse kann über einen breiten Bereich je nach gewählter Herstellungsmethode variieren.

Bei Katalysatorvorläufern, die durch Tränkung (Imprägnierung) hergestellt werden, beträgt der Anteil an Trägermaterial an der aktiven Masse in der Regel mehr als 50 Gew.-%, bevorzugt mehr als 75 Gew.-% uns besonders bevorzugt mehr als 85 Gew.-%.

Bei Katalysatorvorläufern, die durch Fällungsreaktionen, wie Mischfällung oder Auffällung, hergestellt werden, beträgt der Anteil an Trägermaterial an der aktiven Masse in der Regel im Bereich von 10 bis 90 Gew.-%, bevorzugt im Bereich von 15 bis 80 Gew.- % und besonders bevorzugt im Bereich von 20 bis 70 Gew.-%.

Bevorzugte Katalysatorvorläufer, die durch Fällungsreaktionen erhalten werden, sind beispielsweise die

in EP-A-963 975 offenbarte Katalysatoren, deren katalytisch aktive Masse vor der Reduktion mit Wasserstoff 22 bis 40 Gew.-% ZrO₂ 1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das molare Ni : Cu-Verhaltnis größer 1 ist, 15 bis 50 Gew.-% sauerstoffhaltige Verbindungen des Kobalts, berechnet als CoO, 0 bis 10 Gew.-% sauerstoffhaltige Verbindungen des Aluminiums und/oder Mangans, berechnet als Al₂O₃ bzw. MnO₂, und keine sauerstoffhaltigen Verbindungen des Molybdans enthält, beispielsweise der in loc. cit., Seite 17, offenbarte Katalysator A mit der Zusammensetzung 33 Gew.-% Zr, berechnet als ZrO₂, 28 Gew.-% Ni, berechnet als NiO, 11 Gew.-% Cu, berechnet als CuO und 28 Gew.-% Co, berechnet als CoO;

in EP-A2-1106600 offenbarte Katalysatoren, deren katalytische aktive Masse vor der Reduktion mit Wasserstoff 22 bis 45 Gew.-% sauerstoffhaltige Verbindungen des Zirkoniums, berechnet als ZrO₂, 1 bis 30 Gew.-% sauerstoffhaltige Verbindungen des Kupfers, berechnet als CuO, 5 bis 50 Gew.-% sauerstoffhaltige Verbindungen des Nickels, berechnet als NiO, wobei das Molverhältnis von Nickel zu Kupfer grösser 1 ist, 5 bis 50 Gew.-% sauerstoffhaltige Verbindungen des Kobalts, berechnet als CoO, 0 bis 5 Gew.-% sauerstoffhaltige Verbindungen des Molybdäns, berechnet als MoO₃, und 0 bis 10 Gew.-% sauerstoffhaltige Verbindungen des Aluminiums und/oder Mangans, berechnet als Al₂O₃ bzw. MnO₂ enthält;

in WO-A-03/076386 offenbarte Katalysatoren, deren katalytische aktive Masse vor der Reduktion mit Wasserstoff 22 bis 40 Gew.-% sauerstoffhaltige Verbindungen des Zirkoniums, berechnet als ZrO₂, 1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das Molverhältnis von Nickel zu Kupfer grösser 1 ist, 15 bis 50 Gew.-% sauerstoffhaltige Verbindungen des Kobalts, berechnet als CoO, und weniger als 1 Gew.-% Alkalimetall, berechnet als Alkalimetalloxid, enthält;

in EP-A-1852182 offenbarte Katalysatoren, die Kobalt auf einem ZnO-Träger enthalten und folgende Partikelgrößenverteilung aufweisen: < 10 % der Partikel weisen eine Partikelgröße unterhalb einem µm auf, 70 - 99 % der Partikel weisen eine Partikelgröße zwischen 1 und 5 µm auf und < 20 % der Partikel weisen eine Partikelgröße von mehr als 5 µm auf.

Die Katalysatorvorläufer, die kein Trägermaterial enthalten, als auch die Katalysatorvorläufer, die Trägermaterial enthalten, können nach bekannten Verfahren, z.B. durch Fällung, Auffällung oder Imprägnierung hergestellt werden.

In einer bevorzugten Ausführungsform werden Katalysatorvorläufer in das erfindungsgemäße Verfahren eingesetzt, die durch Tränkung (Imprägnierung) von Trägermaterialien hergestellt werden (getränkte Katalysatorvorläufer).

Die Trägermaterialien, die bei der Imprägnierung verwendet werden, können beispielsweise in Form von Pulvern oder Formkörpern, wie Strängen, Tabletten, Kugeln oder Ringen, eingesetzt werden. Für Wirbelbettreaktoren geeignetes Trägermaterial wird vorzugsweise durch Sprühtrocknung erhalten.

Als Trägermaterialien kommen beispielsweise Kohlenstoff, wie Graphit, Russ und/oder Aktivkohle, Aluminiumoxid (gamma, delta, theta, alpha, kappa, chi oder Mischungen daraus), Siliziumdioxid, Zirkoniumdioxid, Zeolithe, Alumosilicate oder deren Gemische in Betracht.

Die Tränkung der obengenannten Trägermaterialien kann nach den üblichen Verfahren erfolgen (A. B. Stiles, Catalyst Manufacture - Laboratory and Commercial Preperations, Marcel Dekker, New York, 1983), beispielsweise durch Aufbringung einer Metallsalzlösung in einer oder mehreren Tränkstufen. Als Metallsalze kommen in der Regel wasserlösliche Metallsalze, wie die Nitrate, Acetate oder Chloride der entsprechenden katalytisch aktiven Komponenten oder Dotierelemente in Betracht, wie Co-Nitrat oder Co-Chlorid. Im Anschluss wird das getränkte Trägermaterial in der Regel getrocknet und ggf. calciniert.

Die Tränkung kann auch nach der sogenannten "incipient wetness-Methode" erfolgen, bei der das Trägermaterial entsprechend seiner Wasseraufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und ggf. zu calcinieren. Die mehrstufige Tränkung ist vorteilhaft dann anzuwenden, wenn das Trägermaterial in größerer Menge mit Metallsalzen beaufschlagt werden soll.

Zur Aufbringung mehrerer Metallkomponenten auf das Trägermaterial, kann die Tränkung gleichzeitig mit allen Metallsalzen oder in beliebiger Reihenfolge der einzelnen Metallsalze nacheinander erfolgen.

In einer weiteren bevorzugten Ausführungsform werden Katalysatorvorläufer über eine gemeinsame Fällung (Mischfällung) aller ihrer Komponenten hergestellt. Dazu wird in der Regel ein lösliches Verbindung der entsprechenden aktiven Komponente, der Dotierelemente und ggf. eine lösliche Verbindung eines Trägermaterials in einer Flüssigkeit in der Wärme und unter Rühren so lange mit einem Fällungsmittel versetzt, bis die Fällung vollständig ist.

Als Flüssigkeit wird in der Regel Wasser eingesetzt.

Als lösliche Verbindung der aktiven Komponenten kommen üblicherweise die entsprechenden Metallsalze, wie die Nitrate, Sulfate, Acetate oder Chloride, der Metalle der Gruppen 8 und/oder 9 und/oder 10 und/oder 11 des Periodensystems der Elemente (Periodensystem in der IUPAC-Fassung vom 22.06.2007), insbesondere von Co, in Betracht.

Als wasserlösliche Verbindungen eines Trägermaterials werden in der Regel wasserlösliche Verbindungen von Ti, Al, Zr, Si etc., beispielsweise die wasserlöslichen Nitrate, Sulfate, Acetate oder Chloride dieser Elemente, verwendet.

Als wasserlösliche Verbindungen der Dotierelemente werden in der Regel wasserlösliche Verbindungen der Dotierelemente, beispielsweise die wasserlöslichen Nitrate, Sulfate, Acetate oder Chloride dieser Elemente, eingesetzt.

Katalysatorvorläufer können weiterhin durch Auffällung hergestellt werden.

Unter Auffällung wird eine Herstellmethode verstanden, bei der ein schwer lösliches oder unlösliches Trägermaterial in einer Flüssigkeit suspendiert wird und nachfolgend lösliche Verbindungen, wie lösliche Metallsalze, der entsprechenden Metalloxide zugegeben werden, welche dann durch Zugabe eines Fällungsmittels auf den suspendierten Träger aufgefällt werden (z.B. beschrieben in EP-A2-1 106 600, Seite 4, und A. B. Stiles, Catalyst Manufacture, Marcel Dekker, Inc., 1983, Seite 15).

Als schwer- bzw. unlösliche Trägermaterialien kommen beispielsweise Kohlenstoffverbindungen, wie Graphit, Russ und/oder Aktivkohle, Aluminiumoxid (gamma, delta, theta, alpha, kappa, chi oder Mischungen daraus), Siliziumdioxid, Zirkoniumdioxid, Zeolithe, Alumosilicate oder deren Gemische in Betracht.

Das Trägermaterial liegt in der Regel als Pulver oder Splitt vor.

Als Flüssigkeit, in der das Trägermaterial suspendiert wird, wird üblicherweise Wasser eingesetzt.

Als lösliche Verbindungen kommen die voranstehend genannten löslichen Verbindungen der aktiven Komponenten bzw. der Dotierelemente in Betracht.

Bei den Fällungsreaktionen ist im Allgemeinen die Art der verwendeten löslichen Metallsalze nicht kritisch. Da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zur Störungen führen, sei es, indem sie unerwünschte Fällungsreaktionen verursachen oder indem sie durch Komplexbildung die Fällung erschweren oder verhindern.

Üblicherweise werden bei den Fällungsreaktionen die löslichen Verbindungen durch Zugabe eines Fällungsmittels als schwer- oder unlösliche, basische Salzen gefällt. Als Fällungsmittel werden bevorzugt Laugen, insbesondere Mineralbasen, wie Alkalimetallbasen eingesetzt. Beispiele für Fällungsmittel sind Natriumcarbonat, Natriumhydroxid, Kaliumcarbonat oder Kaliumhydroxid.

Als Fällungsmittel können auch Ammoniumsalze, beispielsweise Ammoniumhalogenide, Ammoniumcarbonat, Ammoniumhydroxid oder Ammoniumcarboxylate eingesetzt werden.

Die Fällungsreaktionen können z.B. bei Temperaturen von 20 bis 100 °C, besonders 30 bis 90 °C, insbesondere bei 50 bis 70 °C, durchgeführt werden.

Die bei den Fällungsreaktionen erhaltenen Niederschläge sind im Allgemeinen chemisch uneinheitlich und enthalten in der Regel Mischungen der Oxide, Oxidhydrate, Hydroxide, Carbonate und/oder Hydrogencarbonate der eingesetzten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge werden üblicherweise verarbeitet, indem sie gewaschen, getrocknet, calciniert und konditioniert werden.

Nach dem Waschen werden die Niederschläge im Allgemeinen bei 80 bis 200°C, vorzugsweise 100 bis 150°C, getrocknet und anschließend calciniert.

Die Calcinierung wird im Allgemeinen bei Temperaturen zwischen 300 und 800°C, vorzugsweise 400 bis 600°C, insbesondere bei 450 bis 550°C ausgeführt.

Nach der Calcinierung werden die durch Fällungsreaktionen erhaltenen Katalysatorvorläufer üblicherweise konditioniert.

Die Konditionierung kann beispielsweise dadurch erfolgen, dass man den Fällungskatalysator durch Vermahlen auf eine bestimmte Korngröße einstellt.

Nach der Vermahlung kann der durch Fällungsreaktionen erhaltenen Katalysatorvorläufer mit Formhilfsmitteln wie Graphit, oder Stearinsäure vermischt werden und zu Formkörpern weiterverarbeitet werden.

Gängige Verfahren der Formgebung sind beispielsweise im Ullmann [Ullmann's Encyclopedia Electronic Release 2000, Kapitel: "Catalysis and Catalysts", Seiten 28-32] und von Ertl et al. [Ertl, Knözinger, Weitkamp, Handbook of Heterogenoeous Catalysis, VCH Weinheim, 1997, Seiten 98 ff] beschrieben.

Wie in den genannten Literaturstellen beschrieben, können durch den Prozess der Formgebung Formkörper in jeglicher Raumform, z.B. rund, eckig, länglich oder dergleichen, z.B. in Form von Strängen, Tabletten, Granulat, Kugeln, Zylindern oder Körnern erhalten werden. Gängige Verfahren der Formgebung sind beispielsweise Extrusion, Tablettieren, d.h. mechanisches Verpressen oder Pelletieren, d.h. Kompaktieren durch kreisförmige und/oder rotierende Bewegungen.

Nach der Konditionierung bzw. Formgebung erfolgt in der Regel eine Temperung. Die Temperaturen bei der Temperung entsprechen üblicherweise den Temperaturen bei der Calcinierung.

Die durch Fällungsreaktionen erhaltenen Katalysatorvorläufer enthalten die katalytisch aktiven Komponenten in Form eines Gemisches ihrer sauerstoffhaltigen Verbindungen, d.h. insbesondere als Oxide, Mischoxide und/oder Hydroxide. Die so hergestellten Katalysatorvorläufer können als solche gelagert werden.

Vor ihrem Einsatz als Hydrierkatalysatoren in die Stufe b) werden Katalysatorvorläufer, die wie voranstehend beschrieben durch Imprägnierung oder Fällung erhalten wurden, werden in der Regel nach der Calcinierung bzw. Konditionierung durch Behandlung mit Wasserstoff vorreduziert.

Zur Vorreduktion werden die Katalysatorvorläufer im allgemeinen zunächst bei 150 bis 200°C über einen Zeitraum von 12 bis 20 Stunden einer Stickstoff-WasserstoffAtmosphäre ausgesetzt und anschließend noch bis zu ca. 24 Stunden bei 200 bis 400°C in einer Wasserstoffatmosphäre behandelt. Bei dieser Vorreduktion wird ein Teil der in den Katalysatorvorläufern vorliegenden sauerstoffhaltigen Metallverbindungen zu den entsprechenden Metallen reduziert, so dass diese gemeinsam mit den verschiedenartigen Sauerstoffverbindungen in der aktiven Form des Hydrierkatalysators vorliegen.

In einer bevorzugten Ausführungsform wird die Vorreduktion des Katalysatorvorläufers in demselben Reaktor vorgenommen, in dem anschließend die Stufe b) durchgeführt wird.

Der so gebildete Hydrierkatalysator kann nach der Vorreduktion unter einem Inertgas wie Stickstoff gehandhabt und gelagert werden oder unter einer inerten Flüssigkeit, zum Beispiel einem Alkohol, Wasser oder dem Produkt der jeweiligen Reaktion, für die der Hydrierkatalysator eingesetzt wird. Der Hydrierkatalysator kann nach der Vorreduktion aber auch mit einem Sauerstoff enthaltenden Gasstrom wie Luft oder einem Gemisch von Luft mit Stickstoff passiviert, d. h. mit einer schützenden Oxidschicht versehen werden.

Die Lagerung der Hydrierkatalysatoren, die durch Vorreduktion von Katalysatorvorläufern erhalten wurden, unter inerten Substanzen oder die Passivierung des Hydrierkatalysators ermöglichen eine unkomplizierte und ungefährliche Handhabung und Lagerung des Katalysators. Gegebenenfalls muss der Hydrierkatalysator vor Beginn der eigentlichen Reaktion dann von der inerten Flüssigkeit befreit werden bzw. die Passivierungsschicht z. B. durch Behandlung mit Wasserstoff oder einem Wasserstoff enthaltenden Gas aufgehoben werden.

Der Hydrierkatalysator kann vor seinem Einsatz in die Stufe b) von der inerten Flüssigkeit bzw. Passivierungsschicht befreit werden. Dies geschieht beispielsweise durch die Behandlung des Hydrierkatalysators mit Wasserstoff oder einem Wasserstoff enthaltendem Gas. Bevorzugt wird die Stufe b) direkt nach der Behandlung des Hydrierkatalysators im selben Reaktor vorgenommen, in dem auch die Behandlung des Hydrierkatalysators mit Wasserstoff oder einem Wasserstoff enthaltendem Gas erfolgte.

Katalysatorvorläufer können jedoch auch ohne Vorreduktion in das Verfahren eingesetzt werden, wobei sie dann unter den Bedingungen der in Stufe b) stattfindenden

Hydrierung durch den im Reaktor vorhandenen Wasserstoff reduziert werden, wobei sich der Hydrierkatalysator in der Regel in situ bildet.

Die Durchführung der Stufe b) (Hydrierstufe) kann diskontinuierlich oder bevorzugt kontinuierlich durchführt werden.

Die Durchführung der Stufe b) kann in der Flüssigphase oder in der Gasphase durchgeführt werden. Bevorzugt erfolgt die Durchführung der Stufe b) in der Flüssigphase.

Die Umsetzung des in Stufe a) erhaltenen Reaktionsgemisches mit Wasserstoff und Ammoniak kann in Anwesenheit eines Lösungsmittels erfolgen, wobei Bevorzugt das Lösungsmittel eingesetzt wird, das bereits zuvor in Stufe a) eingesetzt wurde, z.B. in Ethern, wie Methyltertbutylether, Ethyltertbutylether oder Tetrahydrofuran (THF); Alkoholen, wie Methanol, Ethanol oder Isopropanol; Kohlenwasserstoffen, wie Hexan, Heptan oder Raffinatschnitte; Aromaten, wie Toluol; Amide, wie Dimethylformamid oder Dimethylacetamid oder Lactamen, wie N-Methylpyrrolidon, N-Ethylpryrrolidon, N-Methylcaprolactam oder N-Ethylcaprolactam. Als Lösungsmittel kommen auch geeignete Mischungen der zuvor aufgeführten Lösungsmittel in Betracht. Das Lösungsmittel kann in einem Anteil von 5 bis 95 % Gew.-%, bevorzugt 20 bis 70 %, besonders bevorzugt 30 bis 60 %, eingesetzt werden, jeweils bezogen auf das Gesamtgewicht des Reaktionsgemisches aus Stufe a) und Lösungsmittel.

Bevorzugt wird die Umsetzung von Acrolein mit sekundärem Amin ohne einen Zusatz von Lösungsmittel durchgeführt.

Die diskontinuierliche Durchführung der Hydrierstufe (Stufe b)) kann beispielsweise in einem Rührautoklaven, einer Blasensäule oder einem Umlaufreaktor, wie etwa einem Strahlschlaufenreaktor, durchgeführt werden.

Bei der diskontinuierlichen Durchführung der Hydrierstufe wird üblicherweise eine Suspension des Reaktionsgemischs aus Stufe a) und Katalysator und ggf. Lösungsmittel im Reaktor vorgelegt. Um einen hohen Umsatz und hohe Selektivität zu gewährleisten, wird die Suspension des Reaktionsgemischs aus Stufe a) und Katalysator mit Ammoniak üblicherweise gut gemischt werden, z.B. durch einen Turbinenrührer in einem Autoklaven. Das suspendierte Katalysatormaterial kann mit Hilfe gebräuchlicher Techniken eingebracht und wieder abgetrennt werden (Sedimentation, Zentrifugation, Kuchenfiltration, Querstromfiltration). Der Katalysator kann einmal oder mehrmals verwendet werden.

Die Katalysatorkonzentration beträgt vorteilhaft 0,1 bis 50 Gew.-%, bevorzugt 0,5 bis 40 Gew.-%, besonders bevorzugt 1 bis 30 Gew.-%, insbesondere 5 bis 20 Gew.-%, jeweils bezogen auf das Gesamtgewicht der Suspension bestehend aus Reaktionsgemisch aus Stufe a) und Katalysator.

Die mittlere Katalysatorpartikelgröße beträgt vorteilhaft im Bereich von 0,001 bis 1 mm, bevorzugt im Bereich von 0,005 bis 0,5 mm, insbesondere 0,01 bis 0,25 mm.

Bei der diskontinuierlichen Durchführung der Stufe b) beträgt der Druck im Allgemeinen von 1-400 bar, bevorzugt 10 bis 300 bar, besonders bevorzugt 20 bis 250 bar. Die Temperatur beträgt im Allgemeinen von 40 bis 400°C, bevorzugt 50 bis 200°C, besonders bevorzugt 60 bis 150°C, insbesondere 60 bis 120°C.

Die Ausführung der Hydrierstufe erfolgt bevorzugt kontinuierlich, üblicherweise in Druckbehältern oder Druckbehälterkaskaden.

Bei der kontinuierlichen Durchführung der Stufe b) in der Flüssigphase leitet man bevorzugt das Reaktionsgemisch aus Stufe a) inklusive Wasserstoff und Ammoniak über den Katalysator, der sich bevorzugt in einem Festbettreaktor befindet. Es ist sowohl eine Rieselfahrweise als auch eine Sumpffahrweise möglich.

Bei der kontinuierlichen Durchführung der Stufe b) in der Flüssigphase beträgt der Druck im Allgemeinen von 1-400 bar, bevorzugt 10 bis 300 bar, besonders bevorzugt 20 bis 250 bar. Die Temperatur beträgt im Allgemeinen von 40 bis 400°C, bevorzugt 50 bis 200°C, besonders bevorzugt 60 bis 150°C, insbesondere 60 bis 120°C.

Bei der kontinuierlichen Durchführung der Stufe b) in der Gasphase wird das Reaktionsgemisch aus Stufe a) zusammen mit Ammoniak in einem zur Verdampfung ausreichend groß gewählten Gasstrom, in Gegenwart von Wasserstoff über den Katalysator geleitet. Bei der Durchführung der Stufe b) in der Gasphase beträgt der Druck im Allgemeinen von 0,1-400 bar, bevorzugt 1 bis 100 bar, besonders bevorzugt 1 bis 50 bar. Die Temperatur beträgt im Allgemeinen von 40 bis 400°C, bevorzugt 50 bis 200°C, besonders bevorzugt 60 bis 150°C, insbesondere 60 bis 120°C.

Die Katalysatorbelastung bei der kontinuierlichen Durchführung der Stufe b) liegt im Allgemeinen im Bereich von 0,05 bis 20, bevorzugt 0,1 bis 15, besonders bevorzugt 0,2 bis 10 kg Reaktionsgemisch aus Stufe a) pro Liter Katalysator (Schüttvolumen) und Stunde.

Das in Stufe b) erhaltene Reaktionsgemisch enthält N,N-substituiertes-1,3-propendiamin.

Das in Stufe b) erhaltene Reaktionsgemisch kann vor der Weiterverwendung bzw. Weiterverarbeitung aufgearbeitet werden, um das N,N -substituierte-1,3-propandiamin aufzukonzentrieren, beispielsweise durch Destillation oder Rektifikation.

Nicht umgesetzte Edukte, wie sekundäre Amine, Wasserstoff oder Ammoniak, können in das Verfahren zurückgeführt werden.

Erfindungsgemäß hergestelltes DMAPA stellt ein wichtiges Zwischenprodukt für die technische Produktion beispielsweise von Schmierseifen und anderen Detergentiendar. DMAPA dient zudem als Ausgangsprodukt für die Herstellung von Koagulationsmitteln und soll selbst antikorrosive Eigenschaften aufweisen. Es dient auch zur Herstellung von Kammpolymeren, welche als Additiv in der Bauindustrie verwendet werden, und anderen Polymeren mit antimikrobiellen Eigenschaften. Demgemäß betrifft die vorliegende Erfindung auch die Verwendung des erfindungsgemäß erhältlichen DMAPAs in den voranstehend genannten Anwendungsgebieten.

Ein Vorteil der Erfindung liegt in der Bereitstellung eines Verfahrens zur Herstellung von N,N-substituierten-1,3-propandiaminen aus Acrolein, wobei gegenüber dem Stand der Technik eine höhere Selektivität bezogen auf das eingesetzte Acrolein erzielt wird. Weiterhin kann die Bildung von Nebenprodukten, wie 3-Methylpiperidin und N,N,N',N'-substiuierten-1,3-propandiaminen, die bei der Hydrierung der intermediär entstehenden N,N,N',N'-substiuierten-1,3-propendiamine anfallen, reduziert werden. Dadurch, dass die als Zwischenstufe erhaltenen N,N,N',N'-substiuierten-1,3-propendiamine vor der weiteren Umsetzung zu N,N-substituierten-1,3-propandiamin nicht isoliert bzw. aufgereinigt werden müssen, ist das Verfahren leicht zu handhaben und technisch realisierbar. Mit der vorliegenden Erfindung wird eine neue Herstellroute für DMAPA zur Verfügung gestellt, in der Einsatzstoffe verwendet werden, die auf Basis nachwachsender Rohstoffe erhalten werden können.

Die Erfindung wird durch die nachstehenden Beispiele näher erläutert.

### Beispiel 1

In einem 270 ml Autoklaven wurden 67,6 g Dimethylamin (1,5 mol) vorgelegt und unter Kühlung (4°C) 16,8 g Acrolein (0,3 mol) während 60 Minuten zugepumpt. Die Mischung wurde 15 Minuten gerührt. Es wurde eine Probe genommen und mittels Gaschromatographie analysiert. Dann wurde der Inhalt dieses Autoklaven mittels einer Verbindungsleitung durch Aufpressen von Wasserstoff in einen 270 ml-Hochdruck-Autoklaven überführt, der zuvor auf 100°C aufgeheizt worden war und in dem bereits 1,8 g Ra-Co (THF-gewaschen) in 51,0 g NH₃ (3,0 Mol) und 10,8 g Wasser (0,6 mol) vorgelegt waren. Die Temperatur des zweiten Autoklaven wurde bei 100°C gehalten und Wasserstoff auf 60 bar aufgepresst. Dann wurde 2 Stunden hydriert und der Druck durch Zugabe von Wasserstoff gehalten. Nach 2 Stunden wurde eine Probe genommen und mittels Gaschromatographie analysiert. Das Chromatogramm zeigte Vollumsatz von Acrolein und des als Zwischenprodukt auftretenden Enamins an und eine Ausbeute von DMAPA von 91,4%. Die Ausbeute (gemessen in GC-FI%) an Dimethylaminopropanol (DMAPOL) betrug 0,4%. Die Ausbeuten der Verbindungen wurden mittels Gaschromatographie als Flächenprozente (F%) ermittelt.

### Beispiel 2

In einem 270 ml Autoklaven wurden 67,6 g Dimethylamin (1,5 mol) vorgelegt und unter Kühlung (4°C) 16,8 g Acrolein (0,3 mol) während 60 Minuten zugepumpt. Die Mischung wurde 15 Minuten gerührt. Es wurde eine Probe genommen und mittels Gaschromatographie analysiert. Dann wurde der Inhalt dieses Autoklaven mittels einer Verbindungsleitung durch Aufpressen von Wasserstoff in einen 270 ml-Hochdruck-Autoklaven überführt, in dem bereits 1,8 g Ra-Co (THF-gewaschen) in 51,0 g NH₃ (3,0 Mol) und 10,8 g Wasser (0,6 mol) vorgelegt waren. Der zweite Autoklav wurde auf 80°C aufgeheizt und Wasserstoff auf 60 bar aufgepresst. Dann wurde 2 Stunden hydriert und der Druck durch Zugabe von Wasserstoff gehalten. Nach 2 Stunden wurde eine Probe genommen und mittels Gaschromatographie analysiert. Das Chromatogramm zeigte Vollumsatz von Acrolein und des als Zwischenprodukt auftretenden Enamins an und eine Ausbeute an DMAPA von 80,3%. Die Ausbeute von Dimethylaminopropanol (DMAPOL) betrug 13,9%. Die Ausbeuten der Verbindungen wurden mittels Gaschromatographie als Flächenprozente (F%) ermittelt.

### Beispiel 3

In einem 270 ml Autoklaven wurden 33.8 g Dimethylamin (0.75 mol) in 30 g THF vorgelegt und unter Kühlung (4°C) 16,8 g Acrolein (0,3 mol) gelöst in 30 g THFwährend 60 Minuten zugepumpt. Die Mischung wurde 15 Minuten gerührt. Es wurde eine Probe genommen und mittels Gaschromatographie analysiert. Dann wurde der Inhalt dieses Autoklaven mittels einer Verbindungsleitung durch Aufpressen von Wasserstoff in einen 270 ml-Hochdruck-Autoklaven überführt, in dem bereits 1,8 g Ra-Co (THF-gewaschen) in 25,5 g NH₃ (1,5 Mol) vorgelegt waren. Der zweite Autoklav wurde auf 80°C aufgeheizt und Wasserstoff auf 60 bar aufgepresst. Dann wurde 2 Stunden hydriert und der Druck durch Zugabe von Wasserstoff gehalten. Nach 6 Stunden wurde eine Probe genommen und mittels Gaschromatographie analysiert. Das Chromatogramm zeigte Vollumsatz von Acrolein und des als Zwischenprodukt auftretenden Enamins an und eine Ausbeute von DMAPA von 88,7%. Die Ausbeute von Dimethylaminopropanol (DMAPOL) betrug 0,8%. Die Ausbeuten der Verbindungen wurden mittels Gaschromatographie als Flächenprozente (F%) ermittelt.

### Beispiel 4

In einem 270 ml Autoklaven wurden 67,6 g Dimethylamin (1,5 mol) vorgelegt und unter Kühlung (4°C) 16,8 g Acrolein (0,3 mol) während 60 Minuten zugepumpt. Die Mischung wurde 15 Minuten gerührt. Es wurde eine Probe genommen und mittels Gaschromatographie analysiert. Dann wurde der Inhalt dieses Autoklaven mittels einer Verbindungsleitung durch Aufpressen von Wasserstoff in einen 270 ml-Hochdruck-Autoklaven überführt, der zuvor auf 120°C aufgeheizt worden war und in dem bereits 1,8 g Ra-Co (THF-gewaschen) in 51,0 g NH₃ (3,0 Mol) und 10,8 g Wasser (0.6 mol) vorgelegt waren. Die Temperatur des zweiten Autoklav wurde auf 120°C gehalten und Wasserstoff auf 100 bar aufgepresst. Dann wurde 2 Stunden hydriert und der Druck durch Zugabe von Wasserstoff gehalten. Nach 3 Stunden wurde eine Probe genommen und mittels Gaschromatographie analysiert. Das Chromatogramm zeigte Vollumsatz von Acrolein und des als Zwischenprodukt auftretenden Enamins an und eine Ausbeute an DMAPA von 87,8%. Die Ausbeute von Dimethylaminopropanol (DMAPOL) betrug 0,8%. Die Ausbeuten der Verbindungen wurden mittels Gaschromatographie als Flächenprozente (F%) ermittelt.

### Beispiel 5

In einem 270 ml Autoklaven wurden 67,6 g Dimethylamin (1,5 mol) vorgelegt und unter Kühlung (4°C) 16,8 g Acrolein (0,3 mol) während 60 Minuten zugepumpt. Die Mischung wurde 15 Minuten gerührt. Es wurde eine Probe genommen und mittels Gaschromatographie analysiert. Dann wurde der Inhalt dieses Autoklaven mittels einer Verbindungsleitung durch Aufpressen von Wasserstoff in einen 270 ml-Hochdruck-Autoklaven überführt, in dem bereits 1,8 g Ra-Co (THF-gewaschen) in 51,0 g NH₃ (3,0 Mol) und 10.8 g Wasser (0.6 mol) vorgelegt waren. Der zweite Autoklav wurde auf 60°C aufgeheizt und Wasserstoff auf 60 bar aufgepresst. Dann wurde 2 Stunden hydriert und der Druck durch Zugabe von Wasserstoff gehalten. Nach 3 Stunden wurde eine Probe genommen und mittels Gaschromatographie analysiert. Das Chromatogramm zeigte Vollumsatz von Acrolein und des als Zwischenprodukt auftretenden Enamins an und eine Ausbeute von DMAPA von 58,2%. Die Ausbeute von Dimethylaminopropanol (DMAPOL) betrug 33,8%. Die Ausbeuten der Verbindungen wurden mittels Gaschromatographie als Flächenprozente (F%) ermittelt.

### Beispiel 6

In einem 270 ml Autoklaven wurden 67,6 g Dimethylamin (1,5 mol) vorgelegt und unter Kühlung (4°C) 16,8 g Acrolein (0,3 mol) während 60 Minuten zugepumpt. Die Mischung wurde 15 Minuten gerührt. Es wurde eine Probe genommen und mittels Gaschromatographie analysiert. Dann wurde der Inhalt dieses Autoklaven mittels einer Verbindungsleitung durch Aufpressen von Wasserstoff in einen 270 ml-Hochdruck-Autoklaven überführt, in dem bereits 1,8 g Ra-Co (THF-gewaschen) in 51,0 g NH₃ (3,0 Mol) vorgelegt waren. Der zweite Autoklav wurde auf 60°C aufgeheizt und Wasserstoff auf 60 bar aufgepresst. Dann wurde 2 Stunden hydriert und der Druck durch Zugabe von Wasserstoff gehalten. Nach 3 Stunden wurde eine Probe genommen und mittels Gaschromatographie analysiert. Das Chromatogramm zeigte Vollumsatz von Acrolein und des als Zwischenprodukt auftretenden Enamins an und eine Ausbeute von DMAPA von 50,4%. Die Ausbeute von Dimethylaminopropanol (DMAPOL) betrug 29,1 %. Die Ausbeuten der Verbindungen wurden mittels Gaschromatographie als Flächenprozente (F%) ermittelt.

### Beispiel 7

In einem 100 ml Kolben wurden 18,25 g Diethylamin wasserfrei (0,25 mol) und 10 g THF vorgelegt und unter Kühlung (4°C) 5,6 g Acrolein (0,1 mol) in 10 g THF zugetropft. Die Mischung wurde in einen 160 ml-Hochdruck-Autoklaven gefüllt; es wurden 0,6 g ™ Ra-Co (THF-gewaschen) zugegeben, der Autoklav wurde verschlossen und anschließend 8,5 g Ammoniak (0,5 mol) hinzugefügt. Es wurde auf 80°C erhitzt und bei Erreichen der gewünschten Reaktionstemperatur auf 60 bar H₂ aufgepresst. Nach 6 Stunden wurde per gaschromatographischer Analyse festgestellt, dass sich 87% des gewünschten Produktes N,N-Diethyl-1,3-propandiamin gebildet hatten. Es wurde kein 3-Methylpiperidin gefunden.

### Vergleichsbeispiel 7a:

Bei Verwendung von Raney-Nickel in gleicher Menge anstelle von Raney-Kobalt und ansonsten identischen Versuchsbedingungen wie in Beispiel 7 wird eine Selektivität von 81,3% zu N,N-Diethyl-1,3-propandiamin gefunden.

### Vergleichsbeispiel 7b:

Die Versuchsbedingungen entsprachen den Bedingungen des Beispiels 7, mit dem Unterschied, dass lediglich 9,2 g Diethylamin wasserfrei (0,125 mol) anstelle von 18,25 g Diethylamin wasserfrei (0,25 mol) in die Reaktion eingesetzt wurden. Per gaschromatographischer Analyse einer Probe wurde festgestellt, dass sich 52 GC-FI.-% des gewünschten Produktes N,N-Diethyl-1,3-propandiamin gebildet hatten. Daneben wurden 8 GC-FI.-% 3-Methylpiperidin gefunden.

### Beispiel 8:

In einem 270 ml Autoklaven wurden 33,8 g Dimethylamin (0,75 mol) und 30 g THF vorgelegt und unter Kühlung (4°C) 16,8 g Acrolein (0,3 mol) in 30 g THF während 60 Minuten zugepumpt. Die Mischung wurde 15 Minuten gerührt. Es wurde eine Probe genommen und mittels Gaschromatographie analysiert. Dann wurde der Inhalt dieses Autoklaven mittels einer Verbindungsleitung durch Aufpressen von Wasserstoff in einen 270 ml-Hochdruck-Autoklaven überführt, in dem bereits 1,8 g Ra-Co (THF-gewaschen) in 25,5 g NH3 (1,5 Mol) vorgelegt waren. Der zweite Autoklav wurde auf 80°C aufgeheizt und Wasserstoff auf 60 bar aufgepresst. Dann wurde 3 Stunden hydriert und der Druck durch Zugabe von Wasserstoff gehalten. Nach 3 Stunden wurde eine Probe genommen und mittels Gaschromatographie analysiert. Das Chromatogramm zeigte Vollumsatz von Acrolein und des als Zwischenprodukt auftretenden Enamins an und eine Selektivität zu DMAPA von 88,3%.

### Vergleichsbeispiel 8a:

Bei Verwendung von Raney-Nickel in gleicher Menge anstelle von Raney-Kobalt und ansonsten identischen Versuchsbedingungen wie in Beispiel 2 wird eine Selektivität von 57,5% zu DMPA gefunden.

## Patentansprüche

1. Verfahren zur Herstellung von N,N-substiuierten-1,3-propandiamin durch
a) Umsetzung von sekundären Amin mit Acrolein bei einer Temperatur von (-50) bis 100°C und einem Druck von 0,01 bis 300 bar, und
b) Umsetzung des in Stufe a) erhaltenen Reaktionsgemischs mit Wasserstoff und Ammoniak in Gegenwart eines Hydrierkatalysators bei einer Temperatur von 40 bis 400°C und einem Druck von 1 bis 400 bar,
**dadurch gekennzeichnet, dass** in Stufe a) das molare Verhältnis von sekundärem Amin zu Acrolein 2:1 oder mehr beträgt und der in Stufe b) eingesetzte Hydrierkatalysator Kobalt enthält.

2. Verfahren nach Anspruch 1, **dadurch gekennzeichnet, dass** man die Stufe b) in Gegenwart von Wasser durchführt.

3. Verfahren nach mindestens einem der Ansprüche 1 bis 2, **dadurch gekennzeichnet, dass** das molare Verhältnis von Wasser zu eingesetztem Acrolein im Bereich von 0,5:1 bis 10:1 liegt.

4. Verfahren nach mindestens einem der Ansprüche 1 bis 3, **dadurch gekennzeichnet, dass** man die Stufe b) bei einer Temperatur von 50 bis 200°C und bei einem Druck von 20 bis 250 bar durchführt.

5. Verfahren nach mindestens einem der Ansprüche 1 bis 4, **dadurch gekennzeichnet, dass** das molare Verhältnis des in Stufe b) eingesetzten Ammoniaks zu dem in Stufe a) eingesetzten Acrolein 2:1 bis 100:1 beträgt.

6. Verfahren nach mindestens einem der Ansprüche 1 bis 5, **dadurch gekennzeichnet, dass** der Hydrierkatalysator in metallischer Form vorliegt.

7. Verfahren nach Anspruch 6, **dadurch gekennzeichnet, dass** der molare Anteil an Cobalt-Atomen bezogen auf die Summe aller Metallatome im eingesetzten Hydrierkatalysator, der in metallischer Form in das Verfahren eingesetzt wurde, 50 Mol % und mehr beträgt.

8. Verfahren nach mindestens einem der Ansprüche 6 bis 7, **dadurch gekennzeichnet, dass** der Hydrierkatalysator in metallischer Form ein Schwamm- oder Skelettkatalysatoren nach Raney ist.

9. Verfahren nach mindestens einem der Ansprüche 1 bis 5, **dadurch gekennzeichnet, dass** man den Hydrierkatalysator durch Reduktion von Katalysatorvorläufern erhält, die ein oder mehrere katalytisch aktive Komponenten in Form von sauerstoffhaltigen Verbindungen der Gruppen 8 und/oder 9 und/oder 10 und/oder 11 des Periodensystems der Elemente enthalten.

10. Verfahren nach Anspruch 9, **dadurch gekennzeichnet, dass** der molare Anteil an Co-Atomen bezogen auf die Summe aller Metallatome, die in den eingesetzten katalytisch aktiven Komponenten vorhanden sind, 30 Mol % oder mehr beträgt.

11. Verfahren nach mindestens einem der Ansprüche 1 bis 10, **dadurch gekennzeichnet, dass** man das in Stufe a) erhaltene Reaktionsgemisch vor Einsatz in Stufe b) ohne zusätzliche Aufreinigung oder Aufarbeitung einsetzt.

12. Verfahren nach mindestens einem der Ansprüche 1 bis 11, **dadurch gekennzeichnet, dass** man ein sekundäres Amin ausgewählt aus der Gruppe bestehend aus Dimethylamin, Diethylamin, Dipropylamin, Diisopropylamin, Dibutylamin, Diisobutylamin, Dihexylamin und Di-Cyclohexylamin einsetzt.

13. Verfahren nach Anspruch 12, **dadurch gekennzeichnet, dass** man als sekundäres Amin Dimethylamin und/oder Diethylamin einsetzt.

14. Verfahren nach mindestens einem der Ansprüche 1 bis 13, **dadurch gekennzeichnet, dass** das Glycerin ein Glycerin auf Basis nachwachsender Rohstoffe ist.

15. Verfahren zur Herstellung von Schmierseifen und anderen Detergentien, Koagulationsmitteln, Polymeren oder Kammpolymeren, **dadurch gekennzeichnet, dass** man in einer ersten Stufe N,N-Dimethyl-1,3-propandiamin (DMAPA) gemäß Anspruch 1 herstellt und in einer zweiten Stufe das in der ersten Stufe erhaltene N,N-Dimethyl-1,3-propandiamin (DMAPA) in ein Verfahren zur Herstellung von Schmierseifen und anderen Detergentien, Koagulationsmitteln, Polymeren oder Kammpolymeren einsetzt.

## Claims

1. A process for preparing N,N-substituted 1,3-propanediamine by
a) reacting secondary amine with acrolein at a temperature of from (-50) to 100°C, and a pressure of from 0.01 to 300 bar, and
b) reacting the reaction mixture obtained in stage a) with hydrogen and ammonia in the presence of a hydrogenation catalyst at a temperature of from 40 to 400°C and a pressure of from 1 to 400 bar,
wherein the molar ratio of secondary amine to acrolein in stage a) is 2:1 or more and the hydrogenation catalyst used in stage b) comprises cobalt.

2. The process according to claim 1, wherein stage b) is performed in the presence of water.

3. The process according to at least one of claims 1 and 2, wherein the molar ratio of water to acrolein used is in the range from 0.5:1 to 10:1.

4. The process according to at least one of claims 1 to 3, wherein stage b) is performed at a temperature of from 50 to 200°C and at a pressure of from 20 to 250 bar.

5. The process according to at least one of claims 1 to 4, wherein the molar ratio of the ammonia used in stage b) to the acrolein used in stage a) is from 2:1 to 100:1.

6. The process according to at least one of claims 1 to 5, wherein the hydrogenation catalyst is present in metallic form.

7. The process according to claim 6, wherein the molar proportion of cobalt atoms based on the sum of all metal atoms in the hydrogenation catalyst used, which has been used in the process in metallic form, is 50 mol% or more.

8. The process according to at least one of claims 6 and 7, wherein the hydrogenation catalyst in metallic form is a Raney sponge or skeletal catalyst.

9. The process according to at least one of claims 1 to 5, wherein the hydrogenation catalyst is obtained by reducing catalyst precursors which comprise one or more catalytically active components in the form of oxygen compounds of groups 8 and/or 9 and/or 10 and/or 11 of the Periodic Table of the Elements.

10. The process according to claim 9, wherein the molar proportion of cobalt atoms based on the sum of all metal atoms which are present in the catalytically active components used is 30 mol% or more.

11. The process according to at least one of claims 1 to 10, wherein the reaction mixture obtained in stage a), before use in stage b), is used without additional purification or workup.

12. The process according to at least one of claims 1 to 11, wherein a secondary amine selected from the group consisting of dimethylamine, diethylamine, dipropylamine, diisopropylamine, dibutylamine, diisobutylamine, dihexylamine and dicyclohexylamine is used.

13. The process according to claim 12, wherein the secondary amine used is dimethylamine and/or diethylamine.

14. The process according to at least one of the preceding claims 1 to 13, wherein the glycerol is glycerol based on renewable raw materials.

15. A process for producing lubricant soaps and other detergents, coagulants, polymers or comb polymers which comprises, in a first stage, preparing N,N-dimethyl-1,3-propanediamine (DMAPA) according to claim 1 and, in a second stage, using the N,N-dimethyl-1,3-propanediamine (DMAPA) obtained in the first stage in a process for producing lubricant soaps and other detergents, coagulants, polymers or comb polymers.

## Revendications

1. Procédé de fabrication de 1,3-propanediamine N,N-substituée, par
a) mise en réaction d'une amine secondaire avec de l'acroléine à une température de (-50) à 100 °C et une pression de 0,01 à 300 bar, et
b) mise en réaction du mélange réactionnel obtenu à l'étape a) avec de l'hydrogène et de l'ammoniac en présence d'un catalyseur d'hydrogénation à une température de 40 à 400 °C et une pression de 1 à 400 bar,
**caractérisé en ce qu'**à l'étape a), le rapport molaire entre l'amine secondaire et l'acroléine est de 2:1 ou plus et le catalyseur d'hydrogénation utilisé à l'étape b) contient du cobalt.

2. Procédé selon la revendication 1, **caractérisé en ce que** l'étape b) est réalisée en présence d'eau.

3. Procédé selon au moins l'une quelconque des revendications 1 à 2, **caractérisé en ce que** le rapport molaire entre l'eau et l'acroléine utilisée se situe dans la plage allant de 0,5:1 à 10:1.

4. Procédé selon au moins l'une quelconque des revendications 1 à 3, **caractérisé en ce que** l'étape b) est réalisée à une température de 50 à 200 °C et à une pression de 20 à 250 bar.

5. Procédé selon au moins l'une quelconque des revendications 1 à 4, **caractérisé en ce que** le rapport molaire entre l'ammoniac utilisé à l'étape b) et l'acroléine utilisée à l'étape a) est de 2:1 à 100:1.

6. Procédé selon au moins l'une quelconque des revendications 1 à 5, **caractérisé en ce que** le catalyseur d'hydrogénation se présente sous forme métallique.

7. Procédé selon la revendication 6, **caractérisé en ce que** la proportion molaire d'atomes de cobalt par rapport à la somme de tous les atomes métalliques dans le catalyseur d'hydrogénation utilisé, qui est utilisé sous forme métallique dans le procédé, est de 50 % en moles et plus.

8. Procédé selon au moins l'une quelconque des revendications 6 à 7, **caractérisé en ce que** le catalyseur d'hydrogénation sous forme métallique est un catalyseur éponge ou squelette selon Raney.

9. Procédé selon au moins l'une quelconque des revendications 1 à 5, **caractérisé en ce que** le catalyseur d'hydrogénation est obtenu par réduction de précurseurs de catalyseur, qui contiennent un ou plusieurs composants catalytiquement actifs sous la forme de composés oxygénés des groupes 8 et/ou 9 et/ou 10 et/ou 11 du tableau périodique des éléments.

10. Procédé selon la revendication 9, **caractérisé en ce que** la proportion molaire d'atomes Co par rapport à la somme de tous les atomes métalliques présents dans les composants catalytiquement actifs utilisés est de 30 % en moles ou plus.

11. Procédé selon au moins l'une quelconque des revendications 1 à 10, **caractérisé en ce que** le mélange réactionnel obtenu à l'étape a) est utilisé sans purification ou traitement supplémentaire avant l'utilisation dans l'étape b).

12. Procédé selon au moins l'une quelconque des revendications 1 à 11, **caractérisé en ce qu'**une amine secondaire choisie dans le groupe constitué par la diméthylamine, la diéthylamine, la dipropylamine, la diisopropylamine, la dibutylamine, la diisobutylamine, la dihexylamine et la dicyclohexylamine est utilisée.

13. Procédé selon la revendication 12, **caractérisé en ce que** la diméthylamine et/ou la diéthylamine sont utilisées en tant qu'amine secondaire.

14. Procédé selon au moins l'une quelconque des revendications 1 à 13, **caractérisé en ce que** la glycérine est une glycérine à base de matières premières renouvelables.

15. Procédé de fabrication de savons mous et d'autres détergents, coagulants, polymères ou polymères en peigne, **caractérisé en ce que**, lors d'une première étape, de la N,N-diméthyl-1,3-propanediamine (DMAPA) est fabriquée selon la revendication 1 et, lors d'une seconde étape, la N,N-diméthyl-1,3-propanediamine (DMAPA) obtenue lors de la première étape est utilisée dans un procédé de fabrication de savons mous et d'autres détergents, coagulants, polymères ou polymères en peigne.
